# EUROPÄISCHE PATENTANMELDUNG

(11) **EP 1 090 654 A1**
(43) Veröffentlichungstag der Anmeldung: **11.04.2001**
(21) Anmeldenummer: 99119735.1
(22) Anmeldetag: 05.10.1999
(51) Int. Cl.: A61M 11/04, A61M 15/00

(54) **Verfahren und Vorrichtung zum Freisetzen ätherischer Wirkstoffe aus als Salbe vorliegenden Heilmitteln**

(71) Anmelder: Badewien, Reinhard, 26802 Moormerland (DE)
(72) Erfinder: Badewien, Reinhard, 26802 Moormerland (DE)

(57) **Zusammenfassung**

Zum Freisetzen ätherischer Wirkstoffe aus als apothekenpflichtige medizinische Salbe vorliegenden Heilmitteln zur Behandlung von Atemwegserkrankungen wird eine vorbestimmte Heilmittelmenge in einen Heilmittelbehälter eingegeben, der der Einwirkung einer Wärmequelle ausgesetzt und mittels ungeregeltem elektrischen Betrieb der Wärmequelle auf eine vorbestimmte Temperatur erwärmt und auf dieser gehalten wird. Der Behälter wird in einen Träger eingesetzt, derart, daß zwischen Außenwandungen des Behälters und Wärmequelle isolierende Luftspalte vorbestimmter Breite gegeben sind. Die mittels Strahlung und/oder Konvektion erfolgende Wärmeübertragung von der Wärmequelle an die Außenwandung des Behälters wird durch Beeinflussung der Wärmeübertragungsvorgänge (Strahlung und/oder Konvektion) so eingeregelt und eingestellt, daß der Schmelzpunkt der im Behälter befindlichen jeweiligen Salbe nicht überschritten wird.

## Beschreibung

Die Erfindung bezieht sich auf ein Verfahren sowie auf eine Vorrichtung zur Durchführung des Verfahrens zum Freisetzen ätherischer Wirkstoffe aus als apothekenpflichtige medizinische Salbe vorliegenden Heilmitteln zur Behandlung von Atemwegserkrankungen, bei dem eine vorbestimmte Heilmittel-Menge in einen Heilmittel-Behälter eingegeben wird, der der Einwirkung einer Wärmequelle ausgesetzt wird und mittels ungeregeltem elektrischen Betrieb der Wärmequelle auf eine vorhestimmte Temperatur erwärmt und auf der Temperatur gehalten wird.

Aus Salben sind darin enthaltene ätherische Wirkstoffe nicht ohne weiteres freisetzbar, vor allem nicht so rasch in hoher Konzentration freisetzbar, wie es oftmals erwünscht ist, da sie in die im wesentlichen aus Fetten bestehende Grundsubstanz der Salbe eingebunden sind. Die Freisetzung kann erfahrungsgemäß beschleunigt und gesteigert werden, wenn die jeweilige Salbe erwärmt wird, wobei ein Optimum dann erreicht wird, wenn die Temperatur den der Salbe eigenen Schmelzpunkt erreicht.

Der Schmelzpunkt darf andererseits aber auch nicht überschritten werden, denn überhitzte Salben neigen zu unerwünschter Geruchsbildung.

Die auf dem Markt erhältlichen apothekenpflichtigen medizinischen Salben enthalten als Salbengrundlage unterschiedliche Fette mit unterschiedlichen Schmelztemperaturen (Schmelzpunkten). Außerdem wird der Schmelzpunkt einer Salbe auch durch ihre weiteren Inhaltsstoffe mehr oder weniger beeinflußt, z.B. durch den Stearin-Gehalt.

Sollen Salben eingesetzt werden, um deren ätherische Wirkstoffe für Heilbehandlungen von Atemwegserkrankungen zu nutzen, kann eine einfache unkontrollierte Erwärmung folglich nicht immer zum für die jeweils verwendete Salbe optimalen Ergebnis führen. Entweder läuft die Freisetzung zu rasch oder zu langsam ab, wobei die rasche Freisetzung auch noch die Gefahr der Überhitzung der Salbe in sich birgt.

Der Erfindung liegt die Aufgabe zugrunde, ein Verfahren und eine Vorrichtung zur Durchführung des Verfahrens bereitzustellen, mit dem bzw. der sich Salben auf ihre jeweils spezifische optimale Schmelztemperatur erwärmen und auf dieser Schmelztemperatur erwärmt halten lassen, so daß optimale Freisetzung ihrer ätherischen Wirkstoffe gewährleistet ist.

Diese Aufgabe ist verfahrensmäßig durch die Merkmale des Anspruchs 1 gelöst.

Vorteilhafte Weiterbildungen ergeben sich aus den Merkmalen der Unteransprüche 2 bis 5.

Die Vorrichtung zur Durchführung des Verfahrens zeichnet sich erfindungsgemäß durch die Merkmale des Anspruchs 6 aus, wobei vorteilhafte Weiterbildungen und Ausgestaltungen der Vorrichtung in den Unteransprüchen 7 bis 26 angegeben sind.

Der ungeregelte elektrische Betrieb der Wärmequelle führt dazu, daß die über einer z.B. als Heizplatte ausgebildeten Oberfläche der Wärmequelle anstehende Luft erwärmt wird. Die Wärme der Luft wird durch Konvektion auf den Salbe enthaltenden Behälter übertragen. Gleichzeitig wirkt auch Strahlungswärme der Wärmequelle auf den Behälter.

Von dem Abstand zwischen der Wärme abgebenden Oberfläche der Wärmequelle und der zugekehrten Wandung des Behälters hängt es somit ab, welche Temperatur der Salbe sich einstellt, da sich zwischen der ständigen Wärmenachführung und den Wärmeverlusten relativ rasch ein Gleichgewicht einstellen wird, welches gewährleistet, daß die jeweils verwendete Salbe auf eine etwa dem Schmelzpunkt entsprechende Temperatur gebracht und danach auf dieser Temperatur gehalten wird.

Durch Veränderung des Abstandes, d.h. der Breite der mehr oder weniger isolierenden Luftspalte, kann die jeweils zu erreichende und zu haltende Temperatur-Obergrenze erfindungsgemäß eingestellt werden. Dies ist mit relativ einfachen Konstruktionsmitteln zu erreichen.

Die Wiederholbarkeit der Voreinstellung bestimmter salbenspezifischer Temperaturen ist dadurch möglich, daß Markierungsskalen vorgesehen sind, die der jeweiligen Abstandseinstellung bzw. Breiteneinstellung der Luftspalte zugeordnete Temperaturen angeben.

Eine erste konstruktiv einfach Ausführung der Vorrichtung sieht einen Träger für den Behälter vor, der, auf die Wärmequelle gesetzt, den in den Träger eingesetzten Behälter mit der Salbe in einem vorbestimmten Abstand über der Heizplatte der Wärmequelle hält. Die Wärmequelle hat relativ geringe Leistung, z.B. 10 bis 30 W, vorzugsweise 27 W, so daß sie mit Vorteil ungeregelt betrieben werden kann.

Bei dieser Ausführung ist es mit besonderem Vorteil möglich, als Einstellmittel Entlüftungen anzubringen, die der erwärmten Luft im Träger zwischen Heizplatte und Behälter erlaubt, in die Umgehung zu entweichen, so daß der Wärmeinhalt der entwichenen Luft nicht mehr auf den Behälter wirken kann.

Die Entlüftungsöffnungen sind so gestaltet, daß sie sich mehr oder weniger weit schließen lassen, so daß sich die Wärmeverluste durch entweichende Luft einregeln lassen. Die Markierungsskala, an der der eingestellte Öffnungsgrad der Entlüftungsöffnungen ablesbar ist, erleichtert die Wiederholbarkeit der Einstellung auf vorbestimmte Temperaturen, die die im Behälter befindliche Salbe annehmen soll, damit eine optimale Freisetzung der ätherischen Wirkstoffe aus der Salbe gewährleistet ist und damit der Schmelzpunkt der Salbe nicht überschritten wird.

Bei einer zweiten Ausführungsmöglichkeit der Vorrichtung ist es möglich, ein als Wasserbad nutzbares Gefäß, z.B. einen Topf, mit heißem, vorzugsweise kochendem Wasser, zu befüllen und auf eine elektrische Heizplatte einer Wärmequelle zu stellen. Diese Heizplatte verfügt entweder über einen Thermostaten oder, bevorzugt, über derart geringe Leistung, z.B. 27 Watt, daß ein Warmhalten des Wasserbades mit z.B. einer Temperatur von 86 bis 90 Grad Celsius über einen langen Zeitraum, insbesondere mehrere Stunden, gewährleistet ist.

Würde die verwendete Salbe auf die Temperatur des Wasserbades erwärmt, kann es zum unerwünscht schnellen Schmelzen der Salbe mit fast schlagartiger rascher Freisetzung des Gesamtinhaltes an ätherischen Wirkstoffen kommen. Letztendlich würde die geschmolzene Salbe dabei überhitzt sein und oftmals penetranten Zersetzungsgenich entwickeln.

Bei dem erfindungsgemäßen Verfahren wird dies vermieden, weil die isolierenden Luftspalte zwischen einer als Träger für den Behälter mit Salbe dienenden Schwimmwanne, die in das Wasserbad schwimmend eingesetzt wird, und deren Wandungen die Wassertemperatur annehmen, und den Wandungen des Behälters mit Salbeninhalt zum Einen die Überhitzung ausschließen und zum Anderen eine Einhaltung einer vorbestimmten Salbentemperatur im Behälter ermöglichen, da die Breite der isolierenden Luftspalte auf ein jeweils gewünschtes Maß einstellbar ist. Dieses Einstellen erfolgt vorzugsweise beim Einsetzen des Behälters in die Schwimmwanne.

Da gewährleistet ist, daß sich die Wandungen von Schwimmwanne und Behälter nicht berühren, findet eine unerwünschte direkte Wärmeleitung nicht statt; vielmehr wird nur die Luft in den Spalten zwischen den Innenwandungen der Schwimmwanne, insbesondere ihr in das Wasserbad eingetauchter Boden, und die Außenwandungen des Behälters, ebenfalls vorzugsweise sein Boden, erwärmt, wobei die Außenwandungen des Behälters weitaus niedrigere Temperaturen annehmen. Je weiter der Behälter mit der Salbe von der umgehenden Schwimmwanne entfernt ist, um so weniger wird die Salbe erhitzt.

Die Einstellung der Spaltbreite auf ein Maß, bei dem die Salbe auf Schmelztemperatur erwärmt und auf dieser Temperatur gehalten wird, ist mit einfachen Mitteln möglich.

Die jeweils gewünschte Temperatur wird bei dem erfindungsgemäßen Verfahren bei entsprechend eingestellter Luftspaltbreite ständig beibehalten, so daß es zur optimalen Freisetzung der ätherischen Wirkstoffe aus der jeweils verwendeten Salbe über einen langen Zeitraum kommt.

Ausführungsbeispiele der die Vorrichtung betreffenden Erfindung, aus denen sich weitere erfinderische Merkmale ergeben, sind in der Zeichnung dargestellt. Es zeigen:
- Fig.1: eine Seitenansicht eines Behälters im Schnitt,
- Fig.2: eine Draufsicht des Behälters,
- Fig.3: eine Seitenansicht eines Trägers für den Behälter gem. Fig. 1 u. 2 im Schnitt,
- Fig.4: eine Draufsicht des Trägers gemäß Fig.3,
- Fig.5: eine Seitenansicht einer ersten Ausführung einer kompletten betriebsbereiten Vorrichtung im Schnitt, mit Träger gem. Fig.3, darin eingesetztem Behälter gem. Fig. 1 sowie einer als Wärmequelle dienenden elektrischen Heizplatte,
- Fig.6: eine Draufsicht der Vorrichtung gem. Fig. 5,
- Fig.7: eine Seitenansicht einer Schwimmwanne im Schnitt,
- Fig.8: eine Draufsicht der Schwimmwanne gem. Fig.7,
- Fig.9: eine Seitenansicht eines Behälters für Salbe im Schnitt,
- Fig.10: die Draufsicht des Behälters gemäß Fig.9,
- Fig.11: eine Seitenansicht einer zweiten Ausführung einer komplett zusammengesetzten, betriebsbereiten Vorrichtung zur Durchführung des Verfahrens und
- Fig.12: eine Seitenansicht einer Drehhülse zur Einstellung der Spalthreiten der Luftspalte zwischen Schwimmwanne und darin eingesetztem Behälter im Schnitt.

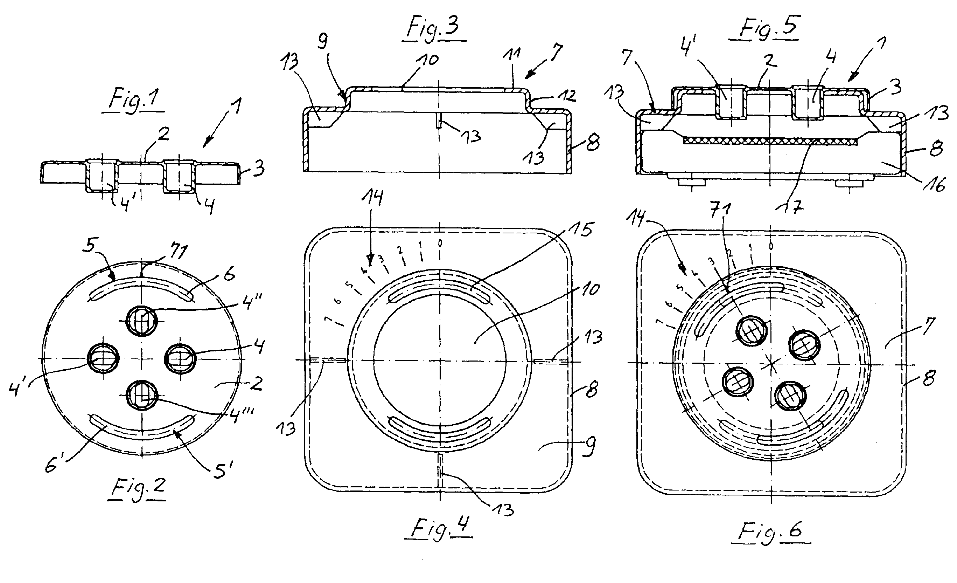

Fig. 1 zeigt in einer Seitenansicht einen Behälter 1, dessen scheibenförmiger Grundkörper 2 einen abgebogenen Außenrand 3 hat, so daß der Behälter 1 in etwa Deckelform hat. In den Grundkörper 2 sind mehrere napfartige Vertiefungen 4, 4' tiefziehähnlich eingeformt, in welche Salbe eingegeben werden kann.

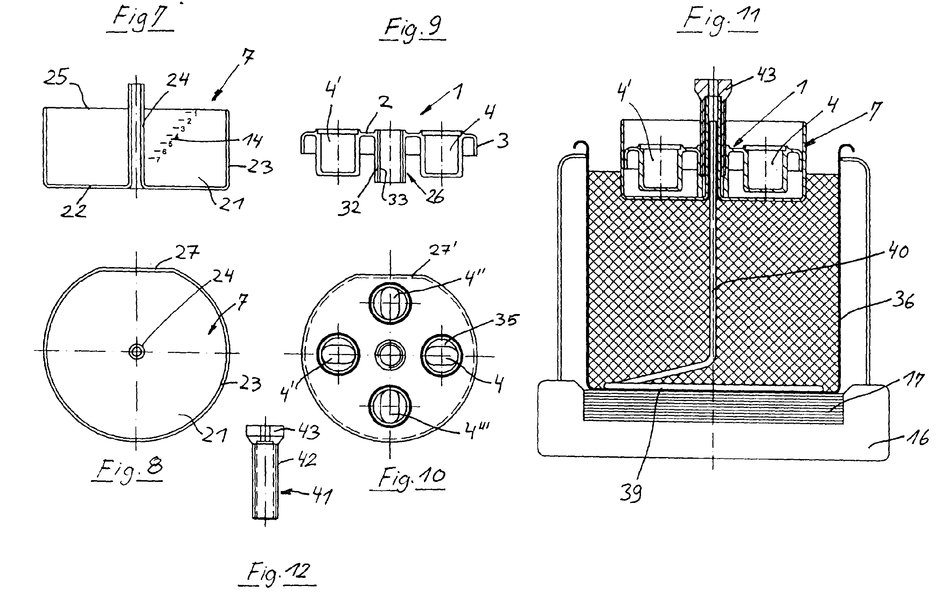

Fig. 2 zeigt eine Draufsicht des Behälters gem. Fig.1, Gleiche Bauteile sind mit gleichen Bezugszahlen bezeichnet. Fig.2 verdeutlicht, daß außer den in Fig. 1 sichtbaren Vertiefungen 4 und 4' noch weitere Vertiefungen 4" und 4''' vorhanden sind. Außerdem weist der scheibenförmige Grundkörper 2 Entlüftungen 5, 5' in Form von hier bohnenförmig gekrümmten Schlitzen 6, 6' auf und eine zum Umfang weisende Pfeilmarkierung 71 zum Anzeigen einer Drehstellung des Behälters gegenüber dem in Fig.3 dargestellten Träger 7.

Fig.2 verdeutlicht desweiteren, daß jede Vertiefung 4, 4', 4", 4"' in ihrem oberen Öffnungsrandbereich rund ist und etwa den Durchmesser eines üblichen Schraubstutzens einer Salbentube aufweist. In dem sich an den Öffnungsrand anschließenden Bereich weist jede Vertiefung 4, 4', 4", 4"' jedoch dann die Form eines Ovals auf, dessen kürzere Achse weniger Lang ist als der Durchmesser des Schraubstutzens einer Tube. Dies vereinfacht das Eingeben von Salbe durch Abdrücken aus einer Tube und es wird die Entstehung von Luftblaseneinschlüssen in der in die Vertiefung eingegebenen Salbe vermieden.

Fig.3 zeigt eine Seitenansicht eines Trägers 7 im Schnitt. Der Träger ist ebenfalls durch herabgezogene Ränder 8 deckel- bzw. haubenartig ausgeformt. In der Mitte seines oberen Wandabschnitts 9 befindet sich eine Öffnung 10, in welche der Behälter 1 einsetzbar ist. Im Seitenbereich der Öffnung 10 sind durch Hochziehen bzw. Herausformen des Wandabschnittes 9 Auflagen 11 bzw. Führungsflächen 12 ausgebildet, auf denen der Behälter 1 aufliegt bzw. mit seinem Außenrand 3 übergreift, sobald er in die Öffnung 10 eingesetzt ist.

Mit 13 sind am als Haubenteil ausgebildeten Träger 7 innen angeformte Abstandhalter bezeichnet.

Fig. 4 zeigt den als Haubenteil ausgebildeten Träger 7 in einer Draufsicht. Gleiche Bauteile sind mit gleichen Bezugszahlen wie in Fig.3 bezeichnet. Fig.4 verdeutlicht, daß der als Haubenteil ausgebildete Träger 7 eine Markierungsskala 14 trägt und ebenfalls Entlüftungsöffnungen 15, 15' aufweist. Die Entlüftungsöffnungen 15, 15' sind zu den Entlüftungsöffnungen 5, 5' im Behälter 1 kongruent, so daß sie je nach Drehstellung des Behälters 1 gegenüber dem Träger 7 mehr oder weniger geschlossen sind, wobei der Öffnungs- bzw. Schließungsgrad an der Markierungsskala 14 mit der Pfeilmarkierung 71 ablesbar ist.

Fig. 5 zeigt eine Wärmequelle 16, die für einen ungeregelten elektrischen Betrieb mit einer Leistung von etwa 10 bis 30 W ausgelegt ist, vorzugsweise 27 W.

Die Wärmequelle 16 verfügt über eine Heizplatte 17.

Der Träger 7 ist an die Wärmequelle 16 angesetzt, wobei er aufgrund seiner Ausbildung als Haubenteil die Wärmequelle 16 außen übergreift. Um dabei den oberen Wandabschnitt 9 von der Heizplatte 17 zu beabstanden, liegt der Träger 7 mit seinen Abstandhaltern 13 auf der Oberseite der Wärmequelle 16 auf. Die untere Fläche des in den Träger 7 eingesetzten Behälters 1, insbesondere die Vertiefungen 4, 4', 4'', 4''' des Behälters 1 befinden sich dabei in einem vorbestimmten Abstand von der Heizplatte 17. Der freie Raum zwischen der Wärmequelle 16 bzw. ihrer Heizplatte 17 und dem Behälter 1 ist ein isolierender Luftspalt vorbestimmter Breite. Durch Konvektion erwärmte Luft kann durch die übereinanderstehenden Entlüftungsöffnungen 5, 5' bzw. 15, 15' entweichen und ist an der Wärmeübertragung an den Behälter 1 nicht mehr beteiligt. Das Entweichen und somit auch die Wärmeübertragung läßt sich durch Öffnen bzw. Schließen der Entlüftungsöffnungen 5, 5'; 15, 15' mittels Verdrehung des Behälters 1 gegenüber dem Träger 7 einstellen bzw. einregeln.

Fig. 6 zeigt in der Draufsicht eine Drehstellung, bei der die Entlüftungsöffnungen etwa halb geschlossen sind, was an der Markierungsskala 14 mit Hilfe der Pfeilmarkierung 71 ablesbar ist.

In Fig.7 ist eine Ausführungsform eines hier als Schwimmwanne 21 ausgebildeten Trägers 7 in einer geschnittenen Seitenansicht dargestellt. Die Schwimmwanne 21 ist napfartig ausgebildet mit einem hier ebenen Boden 22 und geraden Napfwänden 23. In der Mitte des Bodens 22 erhebt sich vom Boden 22 aus in die Schwimmwanne 21 hinein ein Zentralzapfen 24, der hier als Hohlzylinder ausgebildet ist. Der Zentralzapfen 24 steht mit seinem oberen freien Ende etwas über den Öffnungsrand 25 der Schwimmwanne 21 vor.

Die Napfwand 23 ist, wie es hier dargestellt ist, ebenfalls mit einer Markierungsskala 14, beispielsweise mit den hier dargestellten Zahlen Eins bis Sieben, versehen. Die Schwimmwanne 21 besteht vorzugsweise aus transparentem Werkstoff, z.B. Kunststoff, so daß die Markierungsskala 14 auch von Außen durch die Napfwand 23 hindurch sichtbar ist. Selbstverständlich kann die Schwimmwanne 21 auch aus anderen Werkstoffen, z.B. Aluminium oder Keramik, bestehen wobei dann in die Napfwand 23 ein transparentes Fenster eingesetzt sein kann, welches die Markierungsskala 14 trägt.

Fig.8 zeigt die Schwimmwanne 21 in einer Draufsicht. Gleiche Bauelemente sind mit gleichen Bezugszahlen wie in Fig.7 bezeichnet. Fig.8 verdeutlicht, daß die hier kreisförmige Napfwand 23 eine Abplattung aufweist, die Bestandteil einer Verdrehsicherung 27 ist.

In Fig.9 ist eine mögliche Ausführung für einen Behälter 1 in geschnittener Seitenansicht dargestellt. Der Behälter 1 weist ebenfalls vier Vertiefungen 4, 4', 4" 4''' auf, in die Salbe eingebbar ist. Der Behälter 1 besteht im Wesentlichen wiederum aus einem scheibenförmigen Grundkörper 2 mit nach unten abgebogenem Außenrand 3. Im Zentrum des Grundkörpers 2 des Behälters 1 befindet sich eine Führung 26, die hier als Führungshülse 32 ausgebildet ist, welche ein Innengewinde 33 aufweist.

Der gesamte Behälter 1 ist vorzugsweise aus Aluminium oder Keramik hergestellt, da diese Werkstoffe weitgehend resistent sind gegen chemische Reaktionen mit den Salben.

In Fig. 10 ist eine Draufsicht des Behälters 1 dargestellt. Der Außendurchmesser des Behälters 1 ist etwa gleich dem Innendurchmesser der Schwimmwanne 21, dabei jedoch etwas kleiner, so daß der Behälter mit entsprechendem Spiel leichtgängig in die Schwimmwanne 21 einsetzbar und wieder herausnehmbar ist.

Als Verdrehsichening 27' ist wieder ein abgeplatteter Bereich des Außenrands 3 des Behälters 1 vorgesehen, der gewährleistet, daß der Behälter 1 formschlüssig und damit verdrehsicher in der Schwimmwanne 21 aufgenommen ist.

Die Figuren 9 und 10 verdeutlichen weiterhin, daß die Vertiefungen 4, 4', 4" und 4''' in ihrem oberen Öffnungsrandbereich 34 rund sind und dabei etwa den Durchmesser eines üblichen Schraubstutzens einer Salbentube aufweisen. Jede Vertiefung weist dagegen die Form eines Ovales auf, wie es in Fig.10 sichtbar ist. Dadurch ist die Einfüllung von Salben in die Vertiefungen vereinfacht und ist insbesondere gewährleistet, daß die Salbe ohne Luftblaseneinschlüsse und somit die Vertiefung vollständig ausfüllend eingebbar ist, wobei der Schraubstutzen einer Tube während des Abdrückens der Salbe in die ovale Vertiefung auf der Schulter 35 im Übergangsbereich zwischen rundem Öffnungsrandbereich 34 und ovaler Vertiefung abgestützt werden kann.

Fig. 11 zeigt eine Seitenansicht der kompletten, betriebsbereit zusammengesetzten Vorrichtung zur Durchführung des Verfahrens zur Freisetzung ätherischer Wirkstoffe aus Salben. Gleiche Bauteile sind mit gleichen Bezugszahlen bezeichnet.

Mit der in Fig. 11 dargestellten Vorrichtung können ätherische Wirkstoffe über einen langen Zeitraum gleichmäßig freigesetzt werden. Die in die Luft eines Raumes freigesetzten Wirkstoffe könne bei normaler Atmung in die Atemwege gelangen und die gewünschte Heilwirkung entfalten.

Die Vorrichtung umfaßt einen Topf 36, der als Einsatz eines Wärmequelle 16 mit unterer elektrischer Heizplatte 17 ausgebildet ist. Der Topf 36 ist mit kochendem Wasser gefüllt, welches mittels der eingeschalteten Heizplatte 17 konstant warm gehalten wird, bei z.B. 95 Grad Celsius. Auf dem Boden des Topfes 36 steht ein aus Draht gebogener Haltefuß 39, dessen im Zentrum des Topfes 36 hochstehende Stange 40 mit ihrem freien Ende über den oberen Topfrand vorsteht. Auf die Stange 40 ist die Schwimmwanne 21 gesteckt, derart, daß die Stange 40 in dem Zentralzapfen 24 der Schwimmwanne 21 steckt.

In die Vertiefungen 4, 4', 4" und 4"' des Behälters 1 ist Salbe eingefüllt und in die Führungshülse 32 des Behälters 1 ist eine Drehhülse 41 (Fig. 12) geschraubt, die über ein zum Innengewinde 33 der Führungshülse 32 passendes Außengewinde 42 verfügt.

Durch Drehen am Griffknebel 43, der sich am freien Ende der Drehhülse 41 befindet, kann der Abstand zwischen Behälter 1 und dem Boden 22 der Schwimmwanne 21 eingestellt werden, wobei die Markierungsskala 14 benutzt wird, um zur jeweiligen Salbe den jeweils passenden Abstand zu justieren, bei dem sich während des Betriebs der Vorrichtung die Aufrechterhaltung der Temperatur des Schmelzpunktes der verwendeten Salbe ergibt.

Während das Wasser im Topf 36 bei mehrstündigem Betrieb der Vorrichtung langsam verdunstet, kann die Schwimmwanne 21, geführt an der Stange 40 des Haltefußes 39, entsprechend dem sinkenden Wasserspiegel nachgeführt werden.

Die Vorrichtung kann, wie auch bereits vorbeschrieben, trocken, ohne Wasserbad im Topf arbeiten. So kann z.B. die Schwimmwanne 21 durch ein die elektrische Heizplatte 17 übergreifendes Haubenteil als Träger 7 ersetzt sein, in das der Behälter einsetzbar ist. Das Haubenteil kann Innengewinde an lotrechten Haubenseitenwänden aufweisen, so daß das Haubenteil auf ein Außengewinde, das sich z.B. an einen äußeren Kragen der Heizplatte 17 befindet, aufschraubbar ist. Durch die jeweils gewählte Schraubhöhe, abhängig von den Umdrehungen beim Aufschrauben des Haubenteils ergibt sich eine jeweils gewünschte Breite eines Luftspalts zwischen in das Haubenteil eingesetztem Behälter und der Oberfläche der Heizplatte. Der isolierende Luftspalt zwischen der Unterseite des Behälters und der Oberseite der Heizplatte wird um so geringer, und damit die dem Behälter vermittelte Wärme um so größer, je weiter das Haubenteil aufgeschraubt wird.

Eine weitere Einstellmöglichkeit bietet auch eine Ausführung mit schrägen Auflaufflanken zwischen Haubenteil und Behälter, so daß bei einer Drehung des Behälters gegenüber dem dabei feststehenden Haubenteil ein Anheben oder Absenken des Behälters gegenüber dem Haubenteil und damit auch gegenüber der Heizplatte erfolgt.

## Patentansprüche

1. Verfahren zum Freisetzen ätherischer Wirkstoffe aus als apothekenpflichtige medizinische Salbe vorliegenden Heilmitteln zur Behandlung von Atemwegserkrankungen, bei dem eine vorbestimmte Heilmittelmenge in einen Heilmittelbehälter eingegeben wird, der der Einwirkung einer Wärmequelle ausgesetzt wird, und mittels ungeregeltem elektrischen Betrieb der Wärmequelle auf eine vorbestimmte Temperatur erwärmt und auf dieser gehalten wird,
**dadurch gekennzeichnet,**
daß der Behälter (1) in einen Träger (7) eingesetzt wird, derart, daß zwischen Außenwandungen des Behälters (1) und Wärmequelle (16) isolierende Luftspalte vorbestimmter Breite gegeben sind und daß die mittels Strahlung und/oder Konvektion erfolgende Wärmeübertragung von der Wärmequelle (16) an die Außenwandung des Behälters (1) durch Beeinflussung der Wärmeübertragungsvorgänge (Strahlung und/oder Konvektion) so eingeregelt und eingestellt wird, daß der Schmelzpunkt der im Behälter (1) befindlichen jeweiligen Salbe nicht überschritten wird.

2. Verfahren nach Anspruch 1, dadurch gekennzeichnet, daß zur Beeinflussung einer Wämeübertragung durch Konvektion wenigstens ein vorbestimmter Bereich der Luftspalte mehr oder weniger weit zur äußeren Umgebung hin geöffnet bzw. geschlossen wird.

3. Verfahren nach Anspruch 2, gekennzeichnet durch die Verwendung von Einstellmitteln zue Einstellung der Weite von offenen Abgängen der Luftspalte zwecks Freisetzung bzw. Zurückhaltung erwärmter Luft aus den Luftspalten.

4. Verfahren nach Anspruch 1, dadurch gekennzeichnet, daß die Breite der Luftspalte eingestellt wird.

5. Verfahren nach Anspruch 4, gekennzeichnet durch die Verwendung von Einstellmitteln zur Einstellung und Justierung der Breite eines Luftspaltes.

6. Vorrichtung zur Durchführung des Verfahrens nach einem der Ansprüche 1 bis 5, gekennzeichnet durch eine ungeregelt elektrisch betriebene Wärmequelle (16), einen Behälter (1) zur Aufnahme von als apothekenpflichtige medizinische Salbe vorliegendem Heilmittel, durch einen an die Wärmequelle (16) ansetzbaren Träger (7) in den der Behälter (1) einsetzbar ist, und durch Einstellmittel zur Einstellung und Regelung der Wärmeübertragung von der Wärmequelle (16) auf den in den Träger (7) eingesetzten Behälter (1).

7. Vorrichtung nach Anspruch 6, dadurch gekennzeichnet, daß die Wärmequelle (16) ein Standgestell mit einer an seiner Oberseite befindlichen elektrischen Heizplatte (17) aufweist.

8. Vorrichtung nach Anspruch 7, dadurch gekennzeichnet, daß die Heizplatte eine elektrische Leistung von etwa 27 W hat.

9. Vorrichtung nach einem der Ansprüche 6 bis 8, dadurch gekennzeichnet, daß der Behälter (1) einen scheibenförmigen Grundkörper (2) aufweist, in den wenigstens eine Vertiefung (4, 4', 4'', 4''') eingebracht ist, in die Salbe eingebbar ist.

10. Vorrichtung nach einem der Ansprüche 6 bis 9, dadurch gekennzeichnet, daß als Werkstoff für den Behälter (1) Keramik, Glas oder dergl. vorgersehen ist.

11. Vorrichtung nach einem der Ansprüche 6 bis 10, dadurch gekennzeichnet, daß jede Vertiefung (4, 4', 4", 4''') die Form eines Ovals aufweist, dessen kurze Achse etwas weniger Lang ist als der Durchmesser des Schraubstutzens üblicher Salbentuben.

12. Vorrichtung nach einem der Ansprüche 6 bis 11, dadurch gekennzeichnet, daß der Träger (7) als auf die Wärmequelle (16) setzbares Haubenteil ausgebildet ist, dessen sich über der Heizplatte (17) befindlicher Wandabschnitt (9) eine Öffnung (10) hat, in welche der Behälter (1) einsetzbar ist.

13. Vorrichtung nach einem der Ansprüche 6 bis 12, dadurch gekennzeichnet, daß der Haubenteil (Träger 7) und/oder die Wärmequelle(16) und/oder der Behälter (1) Einstellmittel für eine vorbestimmte Breite eines Luftspaltes zwischen eingesetzem Behälter (1) und der Oberseite der Heizplatte (17) der Wärmequelle (16) aufweisen.

14. Vorrichtung nach Anspruch 13, dadurch gekennzeichnet, daß der als Haubenteil ausgebildete Träger (7) auf der Wärmequelle (16) aufliegende Abstandhalter (13) vorbestimmter Höhe aufweist.

15. Vorrichtung nach einem der Ansprüche 13 und 14, dadurch gekennzeichnet daß ein oberer Wandabschnitt (9) des als Haubenteil ausgebildeten Trägers (7) vorkragende Auflageschultern (11) für den eingesetzten Behälter (1) aufweist.

16. Vorrichtung nach einem der Ansprüche 6 bis 15, dadurch gekennzeichnet, daß der Behälter (1) in die Öffnung (10) des als Haubenteil ausgebildeten Trägers (7) drehbar einsetzbar ist.

17. Vorrichtung nach einem der Ansprüche 6 bis 16, dadurch gekennzeichnet, daß die gegenseitigen Auflageflächen von Behälter (1) und Träger (7) zueinander kongruente Durchbrüche (Entlüftungsöffnungen 5, 5'; 15, 15') aufweisen, die je nach Drehstellung des Behälters (1) gegenüber dem Träger (7) mehr oder weniger offen bzw. geschlossen sind.

18. Vorrichtung nach einem der Ansprüche 6 bis 17, dadurch gekennzeichnet, daß der Träger (7) eine Markierungsskala (14) zur Einstellung einer vorbestimmten Drehstellung aufweist, so daß die Variation der Drehstellung die Funktion von Einstellmitteln erfüllt.

19. Vorrichtung nach einem der Ansprüche 6 bis 11, dadurch gekennzeichnet, daß die Heizplatte (17) mit einem Topf (36) zur Aufnahme einer vorbestimmten Menge kochenden Wassers mit einer Temperatur von etwa 95°C kombiniert ist, daß der Träger (7) als oben offene, auf die Wasseroberfläche setzbare Schwimmwanne (21) ausgebildet ist und daß Einstellmittel zur Ausbildung der isolierenden Luftspalte vorbestimmter Breite zwischen einander benachbarten Wänden von Schwimmwanne (21) und Behälter(1) vorgesehen sind.

20. Vorrichtung nach Anspruch 19, dadurch gekennzeichnet, daß die Schwimmwanne (21) einen von ihrem Boden (22) hochstehenden Zentralzapfen (24) aufweist und daß der Behälter (1) für Heilmittel eine den Zentralzapfen (24) umgreifende Führung (26) aufweist.

21. Vorrichtung nach einem der Ansprüche 19 bis 20, dadurch gekennzeichnet, daß die Führung (26) eine etwa im Zentrum des Grundkörpers (2) angeordnete Führungshülse (32) ist.

22. Vorrichtung nach einem der Ansprüche 19 bis 21, dadurch gekennzeichnet, daß der Zentralzapfen (24) mit Außengewinde und die Führungshülse (12) mit auf das Außengewinde schraubbaren Innengewinde (33) ausgerüstet ist.

23. Vorrichtung nach einem der Ansprüche 19 bis 21, dadurch gekennzeichnet, daß eine auf den Zentralzapfen (24) steckbare Drehhülse (41) vorgesehen ist, deren Länge etwa gleich der Länge des Zentralzapfens (24) ist, daß die Drehhülse (41) ein Außengewinde (42) hat und daß die Führungshülse (22) ein Innengewinde (33) hat, in das die Drehhülse (41) einschraubbar ist.

24. Vorrichtung nach einem Ansprüche 19 bis 23, dadurch gekennzeichnet, daß Schwimmwanne (21) und Grundkörper (2) des Behälters (1) mit wenigstens einem Element einer Verdrehsicherung (27, 27') ausgerüstet sind.

25. Vorrichtung nach einem der Ansprüche 19 bis 24, dadurch gekennzeichnet, daß Schwimmwanne (21) und Grundkörper (2) des Behälters (1) kongruente Umrißformen aufweisen und daß als Verdrehsicherung (27, 27') formschlüssig ineinandergreifende Verformungen des Grundrisses vorgesehen sind.

26. Vorrichtung nach einem der Ansprüche 19 bis 25, dadurch gekennzeichnet, daß die Schwimmwanne (21) eine Markierungsskala (14) aufweist, an der die Eintauchtiefe des Behälters (1) in die Schwimmwanne (21) ablesbar ist.
